# EUROPEAN PATENT SPECIFICATION

(11) **EP 1 320 586 B1**
(45) Date of publication and mention of the grant of the patent: **25.08.2004**
(21) Application number: 01972003.6
(22) Date of filing: 05.09.2001
(51) Int. Cl.: C12N 9/02, C12P 13/02, C12N 1/21

(54) **PROCESS FOR THE FERMENTATIVE PREPARATION OF D-PANTOTHENIC ACID USING CORYNEFORM BACTERIA**
PROZESS ZUR FERMENTATIVEN HERSTELLUNG VON D-PANTOTHENSÄURE MIT CORYNEFORMEN BAKTERIEN
PROCEDE DE PREPARATION PAR FERMENTATION D'ACIDE D-PANTOTHENIQUE AU MOYEN DE BACTERIES DE TYPE CORYNEBACTERIE

(30) Priority: 30.09.2000 DE 10048604; 06.04.2001 DE 10117085
(43) Date of publication of application: 25.06.2003
(73) Proprietor: Degussa AG, 40474 Düsseldorf (DE)
(72) Inventor: DUSCH, Nicole, 33824 Werther (DE); HERMANN, Thomas, 33739 Bielefeld (DE); THIERBACH, Georg, 33613 Bielefeld (DE)
(86) International application number: PCT/EP2001/010212
(87) International publication number: WO 2002/029020

(56) References cited:
- EP-A- 0 493 060
- EP-A- 0 590 857
- EP-A- 1 006 189
- CONTIERO ET AL.: "Effects of mutations in acetate metabolism on high-cell-density growth of Escherichia coli" JOURNAL OF INDUSTRIAL MICROBIOLOGY & BIOTECHNOLOGY, vol. 24, no. 6, 2000, page 421-430 XP001041880

### SEQUENCE LISTING

<110> Degussa AG
<120> Process for the fermentative preparation of D-pantothenic acid using coryneform bacteria.
<130> 000439 BT
<140>
   <141>
<160> 12
<170> PatentIn Ver. 2.1
<210> 1
   <211> 2160
   <212> DNA
   <213> Corynebacterium glutamicum
<220>
   <221> CDS
   <222> (327)..(2063)
<220>
   <221> -35_signal
   <222> (227)..(232)
<220>
   <221> -10_signal
   <222> (256)..(261)
<400> 1
   ttagaggcga ttctgtgagg tcactttttg tggggtcggg gtctaaattt ggccagtttt 60
   cgaggcgacc agacaggcgt gcccacgatg tttaaatagg cgatcggtgg gcatctgtgt 120
   ttggtttcga cgggctgaaa ccaaaccaga ctgcccagca acgacggaaa tcccaaaagt 180
   gggcatccct gtttggtacc gagtacccac ccgggcctga aactccctgg caggcgggcg 240
   aagcgtggca acaactggaa tttaagagca caattgaagt cgcaccaagt taggcaacac 300
   aatagccata acgttgagga gttcag atg gca cac agc tac gca gaa caa tta  353
                                Met Ala His Ser Tyr Ala Glu Gln Leu
                                 1               5
   att gac act ttg gaa gct caa ggt gtg aag cga att tat ggt ttg gtg   401
   Ile Asp Thr Leu Glu Ala Gln Gly Val Lys Arg Ile Tyr Gly Leu Val
    10                  15                  20                  25
   ggt gac agc ctt aat ccg atc gtg gat gct gtc cgc caa tca gat att   449
   Gly Asp Ser Leu Asn Pro Ile Val Asp Ala Val Arg Gln Ser Asp Ile
                    30                  35                  40
   gag tgg gtg cac gtt cga aat gag gaa gcg gcg gcg ttt gca gcc ggt    497
   Glu Trp Val His Val Arg Asn Glu Glu Ala Ala Ala Phe Ala Ala Gly
               45                  50                  55
   gcg gaa tcg ttg atc act ggg gag ctg gca gta tgt gct gct tct tgt    545
   Ala Glu Ser Leu Ile Thr Gly Glu Leu Ala Val Cys Ala Ala Ser Cys
           60                  65                  70
   ggt cct gga aac aca cac ctg att cag ggt ctt tat gat tcg cat cga    593
   Gly Pro Gly Asn Thr His Leu Ile Gln Gly Leu Tyr Asp Ser His Arg
       75                  80                  85
   aat ggt gcg aag gtg ttg gcc atc gct agc cat att ccg agt gcc cag    641
   Asn Gly Ala Lys Val Leu Ala Ile Ala Ser His Ile Pro Ser Ala Gln
   90                  95                  100                 105
   att ggt tcg acg ttc ttc cag gaa acg cat ccg gag att ttg ttt aag    689
   Ile Gly Ser Thr Phe Phe Gln Glu Thr His Pro Glu Ile Leu Phe Lys
                   110                 115                 120
   gaa tgc tct ggt tac tgc gag atg gtg aat ggt ggt gag cag ggt gaa    737
   Glu Cys Ser Gly Tyr Cys Glu Met Val Asn Gly Gly Glu Gln Gly Glu
               125                 130                 135
   cgc att ttg cat cac gcg att cag tcc acc atg gcg ggt aaa ggt gtg    785
   Arg Ile Leu His His Ala Ile Gln Ser Thr Met Ala Gly Lys Gly Val
           140                 145                 150
   tcg gtg gta gtg att cct ggt gat atc gct aag gaa gac gca ggt gac    833
   Ser Val Val Val Ile Pro Gly Asp Ile Ala Lys Glu Asp Ala Gly Asp
       155                 160                 165
   ggt act tat tcc aat tcc act att tct tct ggc act cct gtg gtg ttc    881
   Gly Thr Tyr Ser Asn Ser Thr Ile Ser Ser Gly Thr Pro Val Val Phe
   170                 175                 180                 185
   ccg gat cct act gag gct gca gcg ctg gtg gag gcg att aac aac gct    929
   Pro Asp Pro Thr Glu Ala Ala Ala Leu Val Glu Ala Ile Asn Asn Ala
                   190                 195                 200
   aag tct gtc act ttg ttc tgc ggt gcg ggc gtg aag aat gct cgc gcg    977
   Lys Ser Val Thr Leu Phe Cys Gly Ala Gly Val Lys Asn Ala Arg Ala
               205                 210                 215
   cag gtg ttg gag ttg gcg gag aag att aaa tca ccg atc ggg cat gcg    1025
   Gln Val Leu Glu Leu Ala Glu Lys Ile Lys Ser Pro Ile Gly His Ala
           220                 225                 230
   ctg ggt ggt aag cag tac atc cag cat gag aat ccg ttt gag gtc ggc    1073
   Leu Gly Gly Lys Gln Tyr Ile Gln His Glu Asn Pro Phe Glu Val Gly
       235                 240                 245
   atg tct ggc ctg ctt ggt tac ggc gcc tgc gtg gat gcg tcc aat gag    1121
   Met Ser Gly Leu Leu Gly Tyr Gly Ala Cys Val Asp Ala Ser Asn Glu
   250                 255                 260                 265
   gcg gat ctg ctg att cta ttg ggt acg gat ttc cct tat tct gat ttc    1169
   Ala Asp Leu Leu Ile Leu Leu Gly Thr Asp Phe Pro Tyr Ser Asp Phe
                   270                 275                 280
   ctt cct aaa gac aac gtt gcc cag gtg gat atc aac ggt gcg cac att      1217
   Leu Pro Lys Asp Asn Val Ala Gln Val Asp Ile Asn Gly Ala His Ile
                   285                 290                 295
   ggt cga cgt acc acg gtg aag tat ccg gtg acc ggt gat gtt gct gca      1265
   Gly Arg Arg Thr Thr Val Lys Tyr Pro Val Thr Gly Asp Val Ala Ala
               300                 305                 310
   aca atc gaa aat att ttg cct cat gtg aag gaa aaa aca gat cgt tcc      1313
   Thr Ile Glu Asn Ile Leu Pro His Val Lys Glu Lys Thr Asp Arg Ser
       315                 320                 325
   ttc ctt gat cgg atg ctc aag gca cac gag cgt aag ttg agc tcg gtg      1361
   Phe Leu Asp Arg Met Leu Lys Ala His Glu Arg Lys Leu Ser Ser Val
   330                 335                 340                 345
   gta gag acg tac aca cat aac gtc gag aag cat gtg cct att cac cct      1409
   Val Glu Thr Tyr Thr His Asn Val Glu Lys His Val Pro Ile His Pro
                   350                 355                 360
   gaa tac gtt gcc tct att ttg aac gag ctg gcg gat aag gat gcg gtg      1457
   Glu Tyr Val Ala Ser Ile Leu Asn Glu Leu Ala Asp Lys Asp Ala Val
               365                 370                 375
   ttt act gtg gat acc ggc atg tgc aat gtg tgg cat gcg agg tac atc      1505
   Phe Thr Val Asp Thr Gly Met Cys Asn Val Trp His Ala Arg Tyr Ile
           380                 385                 390
   gag aat ccg gag gga acg cgc gac ttt gtg ggt tca ttc cgc cac ggc      1553
   Glu Asn Pro Glu Gly Thr Arg Asp Phe Val Gly Ser Phe Arg His Gly
       395                 400                 405
   acg atg gct aat gcg ttg cct cat gcg att ggt gcg caa agt gtt gat      1601
   Thr Met Ala Asn Ala Leu Pro His Ala Ile Gly Ala Gln Ser Val Asp
   410                 415                 420                 425
   cga aac cgc cag gtg atc gcg atg tgt ggc gat ggt ggt ttg ggc atg      1649
   Arg Asn Arg Gln Val Ile Ala Met Cys Gly Asp Gly Gly Leu Gly Met
                   430                 435                 440
   ctg ctg ggt gag ctt ctg acc gtt aag ctg cac caa ctt ccg ctg aag      1697
   Leu Leu Gly Glu Leu Leu Thr Val Lys Leu His Gln Leu Pro Leu Lys
               445                 450                 455
   gct gtg gtg ttt aac aac agt tct ttg ggc atg gtg aag ttg gag atg      1745
   Ala Val Val Phe Asn Asn Ser Ser Leu Gly Met Val Lys Leu Glu Met
           460                 465                 470
   ctc gtg gag gga cag cca gaa ttt ggt act gac cat gag gaa gtg aat      1793
   Leu Val Glu Gly Gln Pro Glu Phe Gly Thr Asp His Glu Glu Val Asn
       475                 480                 485
   ttc gca gag att gcg gcg gct gcg ggt atc aaa tcg gta cgc atc acc      1841
   Phe Ala Glu Ile Ala Ala Ala Ala Gly Ile Lys Ser Val Arg Ile Thr
   490                 495                 500                 505
   gat ccg aag aaa gtt cgc gag cag cta gct gag gca ttg gca tat cct      1889
   Asp Pro Lys Lys Val Arg Glu Gln Leu Ala Glu Ala Leu Ala Tyr Pro
                   510                 515                 520
   gga cct gta ctg atc gat atc gtc acg gat cct aat gcg ctg tcg atc   1937
   Gly Pro Val Leu Ile Asp Ile Val Thr Asp Pro Asn Ala Leu Ser Ile
               525                 530                 535
   cca cca acc atc acg tgg gaa cag gtc atg gga ttc agc aag gcg gcc   1985
   Pro Pro Thr Ile Thr Trp Glu Gln Val Met Gly Phe Ser Lys Ala Ala
           540                 545                 550
   acc cga acc gtc ttt ggt gga gga gta gga gcg atg atc gat ctg gcc   2033
   Thr Arg Thr Val Phe Gly Gly Gly Val Gly Ala Met Ile Asp Leu Ala
       555                 560                 565
   cgt tcg aac ata agg aat att cct act cca tgatgattga tacacctgct     2083
   Arg Ser Asn Ile Arg Asn Ile Pro Thr Pro
   570                 575
   gttctcattg accgcgagcg cttaactgcc aacatttcca ggatggcagc tcacgccggt 2143
   gcccatgaga ttgccct                                                2160
<210> 2
   <211> 579
   <212> PRT
   <213> Corynebacterium glutamicum
<400> 2
   Met Ala His Ser Tyr Ala Glu Gln Leu Ile Asp Thr Leu Glu Ala Gln
   1               5                   10                  15
   Gly Val Lys Arg Ile Tyr Gly Leu Val Gly Asp Ser Leu Asn Pro Ile
               20                  25                  30
   Val Asp Ala Val Arg Gln Ser Asp Ile Glu Trp Val His Val Arg Asn
           35                  40                  45
   Glu Glu Ala Ala Ala Phe Ala Ala Gly Ala Glu Ser Leu Ile Thr Gly
       50                  55                  60
   Glu Leu Ala Val Cys Ala Ala Ser Cys Gly Pro Gly Asn Thr His Leu
   65                  70                  75                  80
   Ile Gln Gly Leu Tyr Asp Ser His Arg Asn Gly Ala Lys Val Leu Ala
                   85                  90                  95
   Ile Ala Ser His Ile Pro Ser Ala Gln Ile Gly Ser Thr Phe Phe Gln
               100                 105                 110
   Glu Thr His Pro Glu Ile Leu Phe Lys Glu Cys Ser Gly Tyr Cys Glu
           115                 120                 125
   Met Val Asn Gly Gly Glu Gln Gly Glu Arg Ile Leu His His Ala Ile
       130                 135                 140
   Gln Ser Thr Met Ala Gly Lys Gly Val Ser Val Val Val Ile Pro Gly
   145                 150                 155                 160
   Asp Ile Ala Lys Glu Asp Ala Gly Asp Gly Thr Tyr Ser Asn Ser Thr
                   165                 170                 175
   Ile Ser Ser Gly Thr Pro Val Val Phe Pro Asp Pro Thr Glu Ala Ala
               180                 185                 190
   Ala Leu Val Glu Ala Ile Asn Asn Ala Lys Ser Val Thr Leu Phe Cys
           195                 200                 205
   Gly Ala Gly Val Lys Asn Ala Arg Ala Gln Val Leu Glu Leu Ala Glu
       210                 215                 220
   Lys Ile Lys Ser Pro Ile Gly His Ala Leu Gly Gly Lys Gln Tyr Ile
   225                 230                 235                 240
   Gln His Glu Asn Pro Phe Glu Val Gly Met Ser Gly Leu Leu Gly Tyr
                   245                 250                 255
   Gly Ala Cys Val Asp Ala Ser Asn Glu Ala Asp Leu Leu Ile Leu Leu
               260                 265                 270
   Gly Thr Asp Phe Pro Tyr Ser Asp Phe Leu Pro Lys Asp Asn Val Ala
           275                 280                 285
   Gln Val Asp Ile Asn Gly Ala His Ile Gly Arg Arg Thr Thr Val Lys
       290                 295                 300
   Tyr Pro Val Thr Gly Asp Val Ala Ala Thr Ile Glu Asn Ile Leu Pro
   305                 310                 315                 320
   His Val Lys Glu Lys Thr Asp Arg Ser Phe Leu Asp Arg Met Leu Lys
                   325                 330                 335
   Ala His Glu Arg Lys Leu Ser Ser Val Val Glu Thr Tyr Thr His Asn
               340                 345                 350
   Val Glu Lys His Val Pro Ile His Pro Glu Tyr Val Ala Ser Ile Leu
           355                 360                 365
   Asn Glu Leu Ala Asp Lys Asp Ala Val Phe Thr Val Asp Thr Gly Met
       370                 375                 380
   Cys Asn Val Trp His Ala Arg Tyr Ile Glu Asn Pro Glu Gly Thr Arg
   385                 390                 395                 400
   Asp Phe Val Gly Ser Phe Arg His Gly Thr Met Ala Asn Ala Leu Pro
                   405                 410                 415
   His Ala Ile Gly Ala Gln Ser Val Asp Arg Asn Arg Gln Val Ile Ala
               420                 425                 430
   Met Cys Gly Asp Gly Gly Leu Gly Met Leu Leu Gly Glu Leu Leu Thr
           435                 440                 445
   Val Lys Leu His Gln Leu Pro Leu Lys Ala Val Val Phe Asn Asn Ser
       450                 455                 460
   Ser Leu Gly Met Val Lys Leu Glu Met Leu Val Glu Gly Gln Pro Glu
   465                 470                 475                 480
   Phe Gly Thr Asp His Glu Glu Val Asn Phe Ala Glu Ile Ala Ala Ala
                   485                 490                 495
   Ala Gly Ile Lys Ser Val Arg Ile Thr Asp Pro Lys Lys Val Arg Glu
               500                 505                 510
   Gln Leu Ala Glu Ala Leu Ala Tyr Pro Gly Pro Val Leu Ile Asp Ile
           515                 520                 525
   Val Thr Asp Pro Asn Ala Leu Ser Ile Pro Pro Thr Ile Thr Trp Glu
       530                 535                 540
   Gln Val Met Gly Phe Ser Lys Ala Ala Thr Arg Thr Val Phe Gly Gly
   545                 550                 555                 560
   Gly Val Gly Ala Met Ile Asp Leu Ala Arg Ser Asn Ile Arg Asn Ile
                   565                 570                 575
   Pro Thr Pro
<210> 3
   <211> 875
   <212> DNA
   <213> Corynebacterium glutamicum
<400> 3
   tgcgagatgg tgaatggtgg tgagcagggt gaacgcattt tgcatcacgc gattcagtcc 60
   accatggcgg gtaaaggtgt gtcggtggta gtgattcctg gtgatatcgc taaggaagac 120
   gcaggtgacg gtacttattc caattccact atttcttctg gcactcctgt ggtgttcccg 180
   gatcctactg aggctgcagc gctggtggag gcgattaaca acgctaagtc tgtcactttg 240
   ttctgcggtg cgggcgtgaa gaatgctcgc gcgcaggtgt tggagttggc ggagaagatt 300
   aaatcaccga tcgggcatgc gctgggtggt aagcagtaca tccagcatga gaatccgttt 360
   gaggtcggca tgtctggcct gcttggttac ggcgcctgcg tggatgcgtc caatgaggcg 420
   gatctgctga ttctattggg tacggatttc ccttattctg atttccttcc taaagacaac 480
   gttgcccagg tggatatcaa cggtgcgcac attggtcgac gtaccacggt gaagtatccg 540
   gtgaccggtg atgttgctgc aacaatcgaa aatattttgc ctcatgtgaa ggaaaaaaca 600
   gatcgttcct tccttgatcg gatgctcaag gcacacgagc gtaagttgag ctcggtggta 660
   gagacgtaca cacataacgt cgagaagcat gtgcctattc accctgaata cgttgcctct 720
   attttgaacg agctggcgga taaggatgcg gtgtttactg tggataccgg catgtgcaat 780
   gtgtggcatg cgaggtacat cgagaatccg gagggaacgc gcgactttgt gggttcattc 840
   cgccacggca cgatggctaa tgcgttgcct catgc                             875
<210> 4
   <211> 3248
   <212> DNA
   <213> Corynebacterium glutamicum
<220>
   <221> CDS
   <222> (802)..(2538)
<400> 4
   gctctcgcag caacaagagc ccacgcagtt ggagcaaacg cagcaccaag tgaagcgatt 60
   ccgaaaatgc tcaagcccat gaggaacatc cggcggtggc cgattttgtc acccaaagtg 120
   ccggtaccca aaagaaggcc cgccatgagc aggggatatg cgttgatgat ccacaacgct 180
   tgggtttcgg tggctgcgag ctgttcacgc agcagaggga gtgcggtgta gagaatcgag 240
   ttgtctacac cgatcagaaa gagaccaccg ctgataacgg cgaggaaagc ccaacgttgg 300
   gttttcgtag gcgcttgcgc ctgtaaggtt tctgaagtca tggatcgtaa ctgtaacgaa 360
   tggtcggtac agttacaact cttttgttgg tgttttagac cacggcgctg tgtggcgatt 420
   taagacgtcg gaaatcgtag gggactgtca gcgtgggtcg ggttctttga ggcgcttaga 480
   ggcgattctg tgaggtcact ttttgtgggg tcggggtcta aatttggcca gttttcgagg 540
   cgaccagaca ggcgtgccca cgatgtttaa ataggcgatc ggtgggcatc tgtgtttggt 600
   ttcgacgggc tgaaaccaaa ccagactgcc cagcaacgac ggaaatccca aaagtgggca 660
   tccctgtttg gtaccgagta cccacccggg cctgaaactc cctggcaggc gggcgaagcg 720
   tggcaacaac tggaatttaa gagcacaatt gaagtcgcac caagttaggc aacacaatag 780
   ccataacgtt gaggagttca g atg gca cac agc tac gca gaa caa tta att   831
                           Met Ala His Ser Tyr Ala Glu Gln Leu Ile
                           1               5                   10
   gac act ttg gaa gct caa ggt gtg aag cga att tat ggt ttg gtg ggt   879
   Asp Thr Leu Glu Ala Gln Gly Val Lys Arg Ile Tyr Gly Leu Val Gly
                       15                  20                  25
   gac agc ctt aat ccg atc gtg gat gct gtc cgc caa tca gat att gag   927
   Asp Ser Leu Asn Pro Ile Val Asp Ala Val Arg Gln Ser Asp Ile Glu
                   30                  35                  40
   tgg gtg cac gtt cga aat gag gaa gcg gcg gcg ttt gca gcc ggt gcg   975
   Trp Val His Val Arg Asn Glu Glu Ala Ala Ala Phe Ala Ala Gly Ala
           45                  50                  55
   gaa tcg ttg atc act ggg gag ctg gca gta tgt gct gct tct tgt ggt   1023
   Glu Ser Leu Ile Thr Gly Glu Leu Ala Val Cys Ala Ala Ser Cys Gly
       60                  65                  70
   cct gga aac aca cac ctg att cag ggt ctt tat gat tcg cat cga aat   1071
   Pro Gly Asn Thr His Leu Ile Gln Gly Leu Tyr Asp Ser His Arg Asn
   75                  80                  85                  90
   ggt gcg aag gtg ttg gcc atc gct agc cat att ccg agt gcc cag att   1119
   Gly Ala Lys Val Leu Ala Ile Ala Ser His Ile Pro Ser Ala Gln Ile
                   95                  100                 105
   ggt tcg acg ttc ttc cag gaa acg cat ccg gag att ttg ttt aag gaa   1167
   Gly Ser Thr Phe Phe Gln Glu Thr His Pro Glu Ile Leu Phe Lys Glu
               110                 115                 120
   tgc tct ggt tac tgc gag atg gtg aat ggt ggt gag cag ggt gaa cgc   1215
   Cys Ser Gly Tyr Cys Glu Met Val Asn Gly Gly Glu Gln Gly Glu Arg
           125                 130                 135
   att ttg cat cac gcg att cag tcc acc atg gcg ggt aaa ggt gtg tcg    1263
   Ile Leu His His Ala Ile Gln Ser Thr Met Ala Gly Lys Gly Val Ser
       140                 145                 150
   gtg gta gtg att cct ggt gat atc gct aag gaa gac gca ggt gac ggt    1311
   Val Val Val Ile Pro Gly Asp Ile Ala Lys Glu Asp Ala Gly Asp Gly
   155                 160                 165                 170
   act tat tcc aat tcc act att tct tct ggc act cct gtg gtg ttc ccg    1359
   Thr Tyr Ser Asn Ser Thr Ile Ser Ser Gly Thr Pro Val Val Phe Pro
                   175                 180                 185
   gat cct act gag gct gca gcg ctg gtg gag gcg att aac aac gct aag    1407
   Asp Pro Thr Glu Ala Ala Ala Leu Val Glu Ala Ile Asn Asn Ala Lys
               190                 195                 200
   tct gtc act ttg ttc tgc ggt gcg ggc gtg aag aat gct cgc gcg cag    1455
   Ser Val Thr Leu Phe Cys Gly Ala Gly Val Lys Asn Ala Arg Ala Gln
           205                 210                 215
   gtg ttg gag ttg gcg gag aag att aaa tca ccg atc ggg cat gcg ctg    1503
   Val Leu Glu Leu Ala Glu Lys Ile Lys Ser Pro Ile Gly His Ala Leu
       220                 225                 230
   ggt ggt aag cag tac atc cag cat gag aat ccg ttt gag gtc ggc atg    1551
   Gly Gly Lys Gln Tyr Ile Gln His Glu Asn Pro Phe Glu Val Gly Met
   235                 240                 245                 250
   tct ggc ctg ctt ggt tac ggc gcc tgc gtg gat gcg tcc aat gag gcg    1599
   Ser Gly Leu Leu Gly Tyr Gly Ala Cys Val Asp Ala Ser Asn Glu Ala
                   255                 260                 265
   gat ctg ctg att cta ttg ggt acg gat ttc cct tat tct gat ttc ctt    1647
   Asp Leu Leu Ile Leu Leu Gly Thr Asp Phe Pro Tyr Ser Asp Phe Leu
               270                 275                 280
   cct aaa gac aac gtt gcc cag gtg gat atc aac ggt gcg cac att ggt    1695
   Pro Lys Asp Asn Val Ala Gln Val Asp Ile Asn Gly Ala His Ile Gly
           285                 290                 295
   cga cgt acc acg gtg aag tat ccg gtg acc ggt gat gtt gct gca aca    1743
   Arg Arg Thr Thr Val Lys Tyr Pro Val Thr Gly Asp Val Ala Ala Thr
       300                 305                 310
   atc gaa aat att ttg cct cat gtg aag gaa aaa aca gat cgt tcc ttc    1791
   Ile Glu Asn Ile Leu Pro His Val Lys Glu Lys Thr Asp Arg Ser Phe
   315                 320                 325                 330
   ctt gat cgg atg ctc aag gca cac gag cgt aag ttg agc tcg gtg gta    1839
   Leu Asp Arg Met Leu Lys Ala His Glu Arg Lys Leu Ser Ser Val Val
                   335                 340                 345
   gag acg tac aca cat aac gtc gag aag cat gtg cct att cac cct gaa    1887
   Glu Thr Tyr Thr His Asn Val Glu Lys His Val Pro Ile His Pro Glu
               350                 355                 360
   tac gtt gcc tct att ttg aac gag ctg gcg gat aag gat gcg gtg ttt    1935
   Tyr Val Ala Ser Ile Leu Asn Glu Leu Ala Asp Lys Asp Ala Val Phe
           365                 370                 375
   act gtg gat acc ggc atg tgc aat gtg tgg cat gcg agg tac atc gag    1983
   Thr Val Asp Thr Gly Met Cys Asn Val Trp His Ala Arg Tyr Ile Glu
       380                 385                 390
   aat ccg gag gga acg cgc gac ttt gtg ggt tca ttc cgc cac ggc acg    2031
   Asn Pro Glu Gly Thr Arg Asp Phe Val Gly Ser Phe Arg His Gly Thr
   395                 400                 405                 410
   atg gct aat gcg ttg cct cat gcg att ggt gcg caa agt gtt gat cga    2079
   Met Ala Asn Ala Leu Pro His Ala Ile Gly Ala Gln Ser Val Asp Arg
                   415                 420                 425
   aac cgc cag gtg atc gcg atg tgt ggc gat ggt ggt ttg ggc atg ctg    2127
   Asn Arg Gln Val Ile Ala Met Cys Gly Asp Gly Gly Leu Gly Met Leu
               430                 435                 440
   ctg ggt gag ctt ctg acc gtt aag ctg cac caa ctt ccg ctg aag gct    2175
   Leu Gly Glu Leu Leu Thr Val Lys Leu His Gln Leu Pro Leu Lys Ala
           445                 450                 455
   gtg gtg ttt aac aac agt tct ttg ggc atg gtg aag ttg gag atg ctc    2223
   Val Val Phe Asn Asn Ser Ser Leu Gly Met Val Lys Leu Glu Met Leu
       460                 465                 470
   gtg gag gga cag cca gaa ttt ggt act gac cat gag gaa gtg aat ttc    2271
   Val Glu Gly Gln Pro Glu Phe Gly Thr Asp His Glu Glu Val Asn Phe
   475                 480                 485                 490
   gca gag att gcg gcg gct gcg ggt atc aaa tcg gta cgc atc acc gat    2319
   Ala Glu Ile Ala Ala Ala Ala Gly Ile Lys Ser Val Arg Ile Thr Asp
                   495                 500                 505
   ccg aag aaa gtt cgc gag cag cta gct gag gca ttg gca tat cct gga    2367
   Pro Lys Lys Val Arg Glu Gln Leu Ala Glu Ala Leu Ala Tyr Pro Gly
               510                 515                 520
   cct gta ctg atc gat atc gtc acg gat cct aat gcg ctg tcg atc cca    2415
   Pro Val Leu Ile Asp Ile Val Thr Asp Pro Asn Ala Leu Ser Ile Pro
           525                 530                 535
   cca acc atc acg tgg gaa cag gtc atg gga ttc agc aag gcg gcc acc    2463
   Pro Thr Ile Thr Trp Glu Gln Val Met Gly Phe Ser Lys Ala Ala Thr
       540                 545                 550
   cga acc gtc ttt ggt gga gga gta gga gcg atg atc gat ctg gcc cgt    2511
   Arg Thr Val Phe Gly Gly Gly Val Gly Ala Met Ile Asp Leu Ala Arg
   555                 560                 565                 570
   tcg aac ata agg aat att cct act cca tgatgattga tacacctgct          2558
   Ser Asn Ile Arg Asn Ile Pro Thr Pro
                   575
   gttctcattg accgcgagcg cttaactgcc aacatttcca ggatggcagc tcacgccggt 2618
   gcccatgaga ttgccctgcg tccgcatgtg aaaacgcaca aaatcattga aattgcgcag 2678
   atgcaggtcg acgccggtgc ccgagggatc acctgcgcaa ccattggcga ggcggaaatt 2738
   tttgccggcg caggttttac ggacatcttt attgcatatc cgctgtatct aaccgatcat 2798
   gcagtgcaac gcctgaacgc gatccccgga gaaatttcca ttggcgtgga ttcggtagag 2858
   atggcacagg cgacggcggg tttgcgggaa gatatcaagg ctctgattga agtggattcg 2918
   ggacatcgta gaagtggagt cacggcgact gcttcagaat tgagtcagat ccgcgaggcg 2978
   ctgggcagca ggtatgcagg agtgtttact tttcctgggc attcttatgg cccgggaaat 3038
   ggtgagcagg cagcagctga tgagcttcag gctctaaaca acagcgtcca gcgacttgct 3098
   ggcggcctga cttctggcgg ttcctcgccg tctgcgcagt ttacagacgc aatcgatgag 3158
   atgcgaccag gcgtgtatgt gtttaacgat tcccagcaga tcacctcggg agcatgcact 3218
   gagaagcagg tggcaatgac ggtgctgtct                                  3248
<210> 5
   <211> 579
   <212> PRT
   <213> Corynebacterium glutamicum
<400> 5
   Met Ala His Ser Tyr Ala Glu Gln Leu Ile Asp Thr Leu Glu Ala Gln
    1               5                  10                  15
   Gly Val Lys Arg Ile Tyr Gly Leu Val Gly Asp Ser Leu Asn Pro Ile
               20                  25                  30
   Val Asp Ala Val Arg Gln Ser Asp Ile Glu Trp Val His Val Arg Asn
           35                  40                  45
   Glu Glu Ala Ala Ala Phe Ala Ala Gly Ala Glu Ser Leu Ile Thr Gly
       50                  55                  60
   Glu Leu Ala Val Cys Ala Ala Ser Cys Gly Pro Gly Asn Thr His Leu
   65                  70                  75                  80
   Ile Gln Gly Leu Tyr Asp Ser His Arg Asn Gly Ala Lys Val Leu Ala
                   85                  90                  95
   Ile Ala Ser His Ile Pro Ser Ala Gln Ile Gly Ser Thr Phe Phe Gln
               100                 105                 110
   Glu Thr His Pro Glu Ile Leu Phe Lys Glu Cys Ser Gly Tyr Cys Glu
           115                 120                 125
   Met Val Asn Gly Gly Glu Gln Gly Glu Arg Ile Leu His His Ala Ile
       130                 135                 140
   Gln Ser Thr Met Ala Gly Lys Gly Val Ser Val Val Val Ile Pro Gly
   145                 150                 155                 160
   Asp Ile Ala Lys Glu Asp Ala Gly Asp Gly Thr Tyr Ser Asn Ser Thr
                   165                 170                 175
   Ile Ser Ser Gly Thr Pro Val Val Phe Pro Asp Pro Thr Glu Ala Ala
               180                 185                 190
   Ala Leu Val Glu Ala Ile Asn Asn Ala Lys Ser Val Thr Leu Phe Cys
           195                 200                 205
   Gly Ala Gly Val Lys Asn Ala Arg Ala Gln Val Leu Glu Leu Ala Glu
       210                 215                 220
   Lys Ile Lys Ser Pro Ile Gly His Ala Leu Gly Gly Lys Gln Tyr Ile
   225                 230                 235                 240
   Gln His Glu Asn Pro Phe Glu Val Gly Met Ser Gly Leu Leu Gly Tyr
                   245                 250                 255
   Gly Ala Cys Val Asp Ala Ser Asn Glu Ala Asp Leu Leu Ile Leu Leu
               260                 265                 270
   Gly Thr Asp Phe Pro Tyr Ser Asp Phe Leu Pro Lys Asp Asn Val Ala
           275                 280                 285
   Gln Val Asp Ile Asn Gly Ala His Ile Gly Arg Arg Thr Thr Val Lys
       290                 295                 300
   Tyr Pro Val Thr Gly Asp Val Ala Ala Thr Ile Glu Asn Ile Leu Pro
   305                 310                 315                 320
   His Val Lys Glu Lys Thr Asp Arg Ser Phe Leu Asp Arg Met Leu Lys
                   325                 330                 335
   Ala His Glu Arg Lys Leu Ser Ser Val Val Glu Thr Tyr Thr His Asn
               340                 345                 350
   Val Glu Lys His Val Pro Ile His Pro Glu Tyr Val Ala Ser Ile Leu
           355                 360                 365
   Asn Glu Leu Ala Asp Lys Asp Ala Val Phe Thr Val Asp Thr Gly Met
       370                 375                 380
   Cys Asn Val Trp His Ala Arg Tyr Ile Glu Asn Pro Glu Gly Thr Arg
   385                 390                 395                 400
   Asp Phe Val Gly Ser Phe Arg His Gly Thr Met Ala Asn Ala Leu Pro
                   405                 410                 415
   His Ala Ile Gly Ala Gln Ser Val Asp Arg Asn Arg Gln Val Ile Ala
               420                 425                 430
   Met Cys Gly Asp Gly Gly Leu Gly Met Leu Leu Gly Glu Leu Leu Thr
           435                 440                 445
   Val Lys Leu His Gln Leu Pro Leu Lys Ala Val Val Phe Asn Asn Ser
       450                 455                 460
   Ser Leu Gly Met Val Lys Leu Glu Met Leu Val Glu Gly Gln Pro Glu
   465                 470                 475                 480
   Phe Gly Thr Asp His Glu Glu Val Asn Phe Ala Glu Ile Ala Ala Ala
                   485                 490                 495
   Ala Gly Ile Lys Ser Val Arg Ile Thr Asp Pro Lys Lys Val Arg Glu
               500                 505                 510
   Gln Leu Ala Glu Ala Leu Ala Tyr Pro Gly Pro Val Leu Ile Asp Ile
           515                 520                 525
   Val Thr Asp Pro Asn Ala Leu Ser Ile Pro Pro Thr Ile Thr Trp Glu
       530                 535                 540
   Gln Val Met Gly Phe Ser Lys Ala Ala Thr Arg Thr Val Phe Gly Gly
   545                 550                 555                 560
   Gly Val Gly Ala Met Ile Asp Leu Ala Arg Ser Asn Ile Arg Asn Ile
                   565                 570                 575
   Pro Thr Pro
<210> 6
   <211> 475
   <212> DNA
   <213> Corynebacterium glutamicum
<400> 6
   gctctcgcag caacaagagc ccacgcagtt ggagcaaacg cagcaccaag tgaagcgatt 60
   ccgaaaatgc tcaagcccat gaggaacatc cggcggtggc cgattttgtc acccaaagtg 120
   ccggtaccca aaagaaggcc cgccatgagc aggggatatg cgttgatgat ccacaacgct 180
   tgggtttcgg tggctgcgag ctgttcacgc agcagaggga gtgcggtgta gagaatcgag 240
   ttgtctacac cgatcagaaa gagaccaccg ctgataacgg cgaggaaagc ccaacgttgg 300
   gttttcgtag gcgcttgcgc ctgtaaggtt tctgaagtca tggatcgtaa ctgtaacgaa 360
   tggtcggtac agttacaact cttttgttgg tgttttagac cacggcgctg tgtggcgatt 420
   taagacgtcg gaaatcgtag gggactgtca gcgtgggtcg ggttctttga ggcgc      475
<210> 7
   <211> 613
   <212> DNA
   <213> Corynebacterium glutamicum
<400> 7
   gcgtccgcat gtgaaaacgc acaaaatcat tgaaattgcg cagatgcagg tcgacgccgg 60
   tgcccgaggg atcacctgcg caaccattgg cgaggcggaa atttttgccg gcgcaggttt 120
   tacggacatc tttattgcat atccgctgta tctaaccgat catgcagtgc aacgcctgaa 180
   cgcgatcccc ggagaaattt ccattggcgt ggattcggta gagatggcac aggcgacggc 240
   gggtttgcgg gaagatatca aggctctgat tgaagtggat tcgggacatc gtagaagtgg 300
   agtcacggcg actgcttcag aattgagtca gatccgcgag gcgctgggca gcaggtatgc 360
   aggagtgttt acttttcctg ggcattctta tggcccggga aatggtgagc aggcagcagc 420
   tgatgagctt caggctctaa acaacagcgt ccagcgactt gctggcggcc tgacttctgg 480
   cggttcctcg ccgtctgcgc agtttacaga cgcaatcgat gagatgcgac caggcgtgta 540
   tgtgtttaac gattcccagc agatcacctc gggagcatgc actgagaagc aggtggcaat 600
   gacggtgctg tct                                                   613
<210> 8
   <211> 20
   <212> DNA
   <213> Artificial sequence
<220>
   <223> Description of the artificial sequence: Primer pox-del1
<400> 8
   atgaggaaca tccggcggtg 20
<210> 9
   <211> 48
   <212> DNA
   <213> Artificial sequence
<220>
   <223> Description of the artificial sequence: Primer pox-del2
<400> 9
   gagaacagca ggagtatcaa tcatcactga actcctcaac gttatggc 48
<210> 10
   <211> 26
   <212> DNA
   <213> Artificial sequence
<220>
   <223> Description of the artificial sequence: Primer pox-del3
<400> 10
   tgatgattga tacacctgct gttctc 26
<210> 11
   <211> 20
   <212> DNA
   <213> Artificial sequence
<220>
   <223> Description of the artificial sequence: Primer pox-del4
<400> 11
   tcattgccac ctgcttctca 20
<210> 12
   <211> 1422
   <212> DNA
   <213> Corynebacterium glutamicum
<220>
   <221> misc_feature
   <222> (1)..(1422)
   <223> Sequence of the delta poxB allele
<220>
   <221> misc_feature
   <222> (723)..(724)
   Deletion of the coding region of the poxB gene
<400> 12
   atgaggaaca tccggcggtg gccgattttg tcacccaaag tgccggtacc caaaagaagg 60
   cccgccatga gcaggggata tgcgttgatg atccacaacg cttgggtttc ggtggctgcg 120
   agctgttcac gcagcagagg gagtgcggtg tagagaatcg agttgtctac accgatcaga 180
   aagagaccac cgctgataac ggcgaggaaa gcccaacgtt gggttttcgt aggcgcttgc 240
   gcctgtaagg tttctgaagt catggatcgt aactgtaacg aatggtcggt acagttacaa 300
   ctcttttgtt ggtgttttag accacggcgc tgtgtggcga tttaagacgt cggaaatcgt 360
   aggggactgt cagcgtgggt cgggttcttt gaggcgctta gaggcgattc tgtgaggtca 420
   ctttttgtgg ggtcggggtc taaatttggc cagttttcga ggcgaccaga caggcgtgcc 480
   cacgatgttt aaataggcga tcggtgggca tctgtgtttg gtttcgacgg gctgaaacca 540
   aaccagactg cccagcaacg acggaaatcc caaaagtggg catccctgtt tggtaccgag 600
   tacccacccg ggcctgaaac tccctggcag gcgggcgaag cgtggcaaca actggaattt 660
   aagagcacaa ttgaagtcgc accaagttag gcaacacaat agccataaag ttgaggagtt 720
   cagtgatgat tgatacacct gctgttctca ttgaccgcga gcgcttaact gccaacattt 780
   ccaggatggc agctcacgcc ggtgcccatg agattgccct gcgtccgcat gtgaaaacgc 840
   acaaaatcat tgaaattgcg cagatgcagg tcgacgccgg tgcccgaggg atcacctgcg 900
   caaccattgg cgaggcggaa atttttgccg gcgcaggttt tacggacatc tttattgcat 960
   atccgctgta tctaaccgat catgcagtgc aacgcctgaa cgcgatcccc ggagaaattt 1020
   ccattggcgt ggattcggta gagatggcac aggcgacggc gggtttgcgg gaagatatca 1080
   aggctctgat tgaagtggat tcgggacatc gtagaagtgg agtcacggcg actgcttcag 1140
   aattgagtca gatccgcgag gcgctgggca gcaggtatgc aggagtgttt acttttcctg 1200
   ggcattctta tggcccggga aatggtgagc aggcagcagc tgatgagctt caggctctaa 1260
   acaacagcgt ccagcgactt gctggcggcc tgacttctgg cggttcctcg ccgtctgcgc 1320
   agtttacaga cgcaatcgat gagatgcgac caggcgtgta tgtgtttaac gattcccagc 1380
   agatcacctc gggagcatgc actgagaagc aggtggcaat ga                    1422

## Description

### Field of the Invention

The invention provides a process for the fermentative preparation of D-pantothenic acid using coryneform bacteria in which the poxB gene is attenuated.

### Prior Art

Pantothenic acid is a vitamin of commercial importance which is used in human medicine and in the pharmaceuticals industry, in the foodstuffs industry and very particularly in animal nutrition.

Pantothenic acid can be prepared by chemical synthesis, or biotechnologically by fermentation of suitable microorganisms in suitable nutrient solutions. In the chemical synthesis, DL-pantolactone is an important intermediate stage. It is prepared in a multi-stage process from formaldehyde, isobutylaldehyde and cyanide. In further process steps, the racemic mixture is separated, D-pantolactone is subjected to a condensation reaction with β-alanine, and the desired D-pantothenic acid is obtained in this way.

The advantage of the fermentative preparation by microorganisms lies in the direct formation of the desired stereoisomeric D-form, which is free from L-pantothenic acid.

Various types of bacteria, such as e.g. Escherichia coli (E. coli), Arthrobacter ureafaciens, Corynebacterium erythrogenes, Brevibacterium ammoniagenes, and also yeasts, such as e.g. Debaromyces castellii, can produce D-pantothenic acid in a nutrient solution which comprises glucose, DL-pantoic acid and β-alanine, as shown in EP-A 0 493 060. EP-A 0 493 060 furthermore shows that in the case of E. coli, the formation of D-pantothenic acid is improved by amplification of pantothenic acid biosynthesis genes from E. coli which are contained on the plasmids pFV3 and pFV5 in a nutrient solution comprising glucose, DL-pantoic acid and β-alanine.

EP-A 0 590 857 and US Patent 5,518,906 describe mutants derived from E. coli strain IFO3547, such as FV5714, FV525, FV814, FV521, FV221, FV6051 and FV5069, which carry resistances to various antimetabolites, such as salicylic acid, α-ketobutyric acid, β-hydroxyaspartic acid, O-methylthreonine and α-ketoisovaleric acid. They produce pantoic acid in a nutrient solution comprising glucose, and D-pantothenic acid in a nutrient solution comprising glucose and β-alanine. It is furthermore shown in EP-A 0 590 857 and US Patent 5,518,906 that after amplification of the pantothenic acid biosynthesis genes contained on the plasmid pFV31, in the above-mentioned strains the production of D-pantoic acid in nutrient solutions comprising glucose and the production of D-pantothenic acid in a nutrient solution comprising glucose and β-alanine is improved.

Processes for the preparation of D-pantothenic acid with the aid of Corynebacterium glutamicum (C. glutamicum) are known only in some instances in the literature. Sahm and Eggeling (Applied and Environmental Microbiology 65(5), 1973-1979 (1999)) thus report on the influence of over-expression of the panB and panC genes and Dusch et al. (Applied and Environmental Microbiology 65(4), 1530-1539 (1999)) report on the influence of the panD gene on the formation of D-pantothenic acid.

### Object of the Invention

The inventors had the object of providing new principles for improved processes for the fermentative preparation of pantothenic acid with coryneform bacteria.

### Summary of the Invention

When D-pantothenic acid or pantothenic acid or pantothenate are mentioned in the following text, this means not only the free acids but also the salts of D-pantothenic acid, such as e.g. the calcium, sodium, ammonium or potassium salt.

The invention provides a process for the fermentative preparation of D-pantothenic acid using coryneform bacteria in which the nucleotide sequence which codes for the enzyme pyruvate oxidase (EC 1.2.2.2) (poxB gene) is attenuated.

This invention also provides a process for the fermentative preparation of D-pantothenic acid, in which the following steps are carried out:
a) fermentation of D-pantothenic acid-producing coryneform bacteria in which at least the nucleotide sequence which codes for pyruvate oxidase (EC 1.2.2.2) (poxB) is attenuated, in particular eliminated;
b) concentration of the D-pantothenic acid in the medium or in the cells of the bacteria; and
c) isolation of the D-pantothenic acid produced.

The strains employed optionally already produce D-pantothenic acid before attenuation of the poxB gene.

Preferred embodiments are to be found in the claims.

### Detailed Description of the Invention

The term "attenuation" in this connection describes the reduction or elimination of the intracellular activity of one or more enzymes (proteins) in a microorganism which are coded by the corresponding DNA, for example by using a weak promoter or using a gene or allele which codes for a corresponding enzyme (protein) with a low activity or inactivates the corresponding gene or enzyme (protein), and optionally combining these measures.

By attenuation measures, the activity or concentration of the corresponding protein is in general reduced to 0 to 75%, 0 to 50%, 0 to 25%, 0 to 10% or 0 to 5% of the activity or concentration of the wild-type protein or of the activity or concentration of the protein in the starting microorganism.

The microorganisms which the present invention provides can produce D-pantothenic acid from glucose, sucrose, lactose, fructose, maltose, molasses, starch, cellulose or from glycerol and ethanol. They are representatives of coryneform bacteria, in particular of the genus Corynebacterium. Of the genus Corynebacterium, there may be mentioned in particular the species Corynebacterium glutamicum, which is known among experts for its ability to produce L-amino acids.

Suitable strains of the genus Corynebacterium, in particular of the species Corynebacterium glutamicum, are, for example, the known wild-type strains
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
also the strains Brevibacterium flavum ATCC14067
Brevibacterium lactofermentum ATCC13869 and
Brevibacterium divaricatum ATCC14020
and D-pantothenic acid-producing mutants prepared therefrom, such as, for example
Corynebacterium glutamicum ATCC13032ΔilvA/pEC7panBC
Corynebacterium glutamicum ATCC13032/pND-D2.

It has been found that coryneform bacteria produce pantothenic acid in an improved manner after attenuation of the poxB gene, which codes for pyruvate oxidase (EC 1.2.2.2).

"Isolated" means separated out of its natural environment.

"Polynucleotide" in general relates to polyribonucleotides and polydeoxyribonucleotides, it being possible for these to be non-modified RNA or DNA or modified RNA or DNA.

The nucleotide sequence of the poxB gene is shown in SEQ ID No. 1 and the resulting amino acid sequence of the enzyme protein is shown in SEQ ID No. 2.

The poxB gene described in SEQ ID No. 1 can be used according to the invention. Alleles of the poxB gene which result from the degeneracy of the genetic code or due to "sense mutations" of neutral function can furthermore be used.

A new nucleotide sequence, shown in SEQ ID No. 6, which lies upstream of the nucleotide sequence of the poxB gene region shown in SEQ ID No. 1 has been found. A new nucleotide sequence, shown in SEQ ID No. 7, which lies downstream of the nucleotide sequence of the poxB gene region shown in SEQ ID No. 1 has furthermore been found. The sequence of the poxB gene region shown in SEQ ID No. 4 has been obtained in this manner.

It has been found that these polynucleotides shown in SEQ ID No. 6 and 7 are useful in the production of mutants with an attenuated, in particular eliminated, poxB gene.

It has also been found that coryneform bacteria produce pantothenic acid in an improved manner after attenuation of the poxB gene.

To achieve an attenuation, either the expression of the poxB gene or the catalytic properties of the enzyme protein can be reduced or eliminated. The two measures can optionally be combined.

The decrease in gene expression can take place by suitable culturing or by genetic modification ("mutation") of the signal structures of gene expression. Signal structures of gene expression are, for example, repressor genes, activator genes, operators, promoters, attenuators, ribosome binding sites, the start codon and terminators. The expert can find information on this e.g. in the patent application WO 96/15246, in Boyd and Murphy (Journal of Bacteriology 170: 5949 (1988)), in Voskuil and Chambliss (Nucleic Acids Research 26: 3548 (1998), in Jensen and Hammer (Biotechnology and Bioengineering 58: 191 (1998)), in Patek et al. (Microbiology 142: 1297 (1999)) and in known textbooks of genetics and molecular biology, such as e.g. the textbook by Knippers ("Molekulare Genetik", 6th edition, Georg Thieme Verlag, Stuttgart, Germany, 1995) or that by Winnacker ("Gene und Klone", VCH Verlagsgesellschaft, Weinheim, Germany, 1990).

Mutations which lead to a change or reduction in the catalytic properties of enzyme proteins are known from the prior art. Examples which may be mentioned are the works of Qiu and Goodman (Journal of Biological Chemistry 272: 8611-8617 (1997)), Sugimoto et al. (Bioscience Biotechnology and Biochemistry 61: 1760-1762 (1997)) and Möckel ("Die Threonindehydratase aus Corynebacterium glutamicum: Aufhebung der allosterischen Regulation und Struktur des Enzyms", Reports from the Jülich Research Center, Jül-2906, ISSN09442952, Jülich, Germany, 1994). Summarizing descriptions can be found in known textbooks of genetics and molecular biology, such as e.g. that by Hagemann ("Allgemeine Genetik", Gustav Fischer Verlag, Stuttgart, 1986).

Possible mutations are transitions, transversions, insertions and deletions. Depending on the effect of the amino acid exchange on the enzyme activity, "missense mutations" or "nonsense mutations" are referred to. Insertions or deletions of at least one base pair (bp) in a gene lead to "frame shift mutations", which lead to incorrect amino acids being incorporated or translation being interrupted prematurely. Deletions of several codons typically lead to a complete loss of the enzyme activity. Instructions on generation of such mutations are prior art and can be found in known textbooks of genetics and molecular biology, such as e.g. the textbook by Knippers ("Molekulare Genetik", 6th edition, Georg Thieme Verlag, Stuttgart, Germany, 1995), that by Winnacker ("Gene und Klone", VCH Verlagsgesellschaft, Weinheim, Germany, 1990) or that by Hagemann ("Allgemeine Genetik", Gustav Fischer Verlag, Stuttgart, 1986).

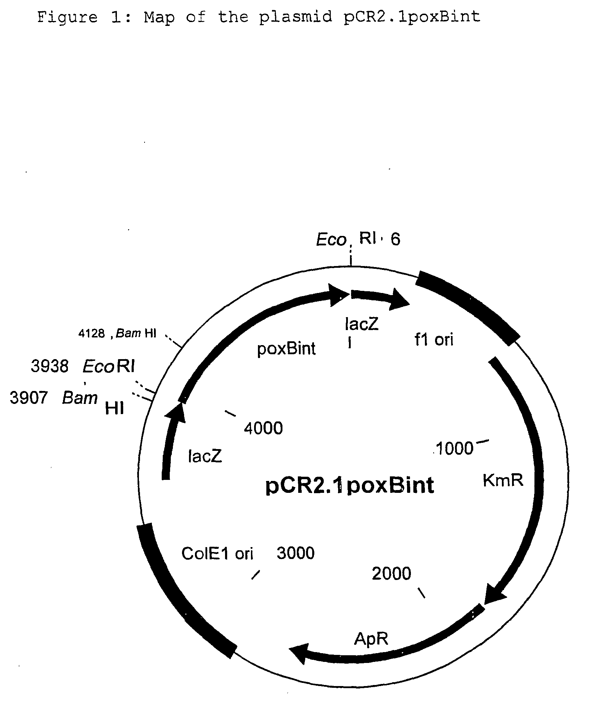

An example of a plasmid with the aid of which an insertion mutagenesis of the poxB gen can be carried out is pCR2.1poxBint (figure 1).

Plasmid pCR2.1poxBint comprises the plasmid pCR2.1-TOPO described by Mead et al. (Bio/Technology 9:657-663 (1991)), into which an internal fragment of the poxB gene, shown in SEQ-ID No. 3 has been incorporated. After transformation and homologous recombination in the chromosomal poxB gene (insertion), this plasmid leads to a total loss of the enzyme function.

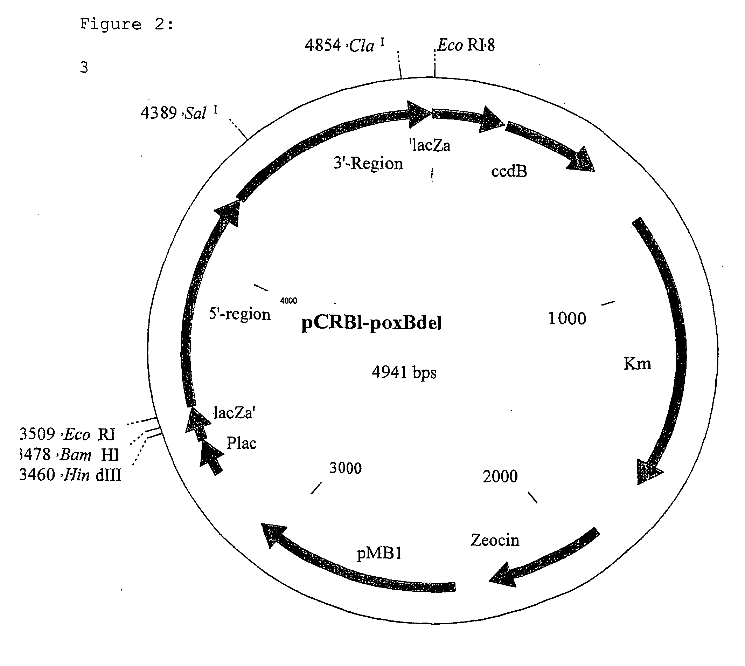

Another example of a mutated poxB gene is the ΔpoxB allele contained in the plasmid pCRB1-poxBdel (figure 2). The ΔpoxB allele contains only the 5' and the 3' flank of the poxB gene. The 1737 bp long section of the coding region is missing (deletion). The nucleotide sequence of the ΔpoxB allele and of the 5' and 3' flank is shown in SEQ ID No. 12. This ΔpoxB allele can be incorporated into coryneform bacteria by integration mutagenesis. The above-mentioned plasmid pCRB1-poxBdel is used for this, or the ΔpoxB allele is transferred to the plasmid pK18mobsacB and the plasmid of the type pK18mobsacBpoxBdel thereby formed is used. After transfer by conjugation or transformation and homologous recombination by means of a first "cross-over" event which effects integration and a second "cross-over" event which effects excision in the poxB gene, the incorporation of the ΔpoxB allele is achieved and a total loss of the enzyme function in the particular strain is achieved. The invention provides the ΔpoxB allele characterized by SEQ ID No. 12.

Further instructions and explanations on insertion mutagenesis or integration mutagenesis and gene replacement are to be found, for example, in Schwarzer and Pühler (Bio/Technology 9,84-87 (1991)), Peters-Wendisch et al. (Microbiology 144, 915-927 (1998)) or Fitzpatrick et al. (Applied Microbiology and Biotechnology 42, 575-580 (1994)).

It may furthermore be advantageous for the production of pantothenic acid, in addition to the attenuation of the gene which codes for pyruvate oxidase, for one or more of the genes chosen from the group consisting of
- the panB gene which codes for ketopantoate hydroxymethyl transferase (Sahm et al., Applied and Environmental Microbiology, 65, 1973-1979 (1999)),
- the panC gene which codes for pantothenate synthetase (Sahm et al., Applied and Environmental Microbiology, 65, 1973-1979 (1999)),
- the ilvC gene which codes for acetohydroxy-acid isomeroreductase (EMBL gene library: Accession No. L09232), and
- the ilvD gene which codes for dihydroxy-acid dehydratase (EP-A-1006189);
to be enhanced, in particular over-expressed.

The term "enhancement" in this connection describes the increase in the intracellular activity of one or more enzymes in a microorganism which are coded by the corresponding DNA, for example by increasing the number of copies of the gene or genes, using a potent promoter or using a gene which codes for a corresponding enzyme having a high activity, and optionally combining these measures.

By attenuation measures, the activity or concentration of the corresponding protein is in general reduced to 0 to 75%, 0 to 50%, 0 to 25%, 0 to 10% or 0 to 5% of the activity or concentration of the wild-type protein or of the activity or concentration of the protein in the starting microorganism.

It may furthermore be advantageous for the production of pantothenic acid, in addition to the attenuation of the gene which codes for pyruvate oxidase, for the pck gene which codes for phosphoenol pyruvate carboxykinase (PEP carboxykinase) (DE: 19950409.1, DSM 13047) to be attenuated.

Finally, in addition to attenuation of pyruvate oxidase, it may be advantageous for the production of pantothenic acid to eliminate undesirable side reactions (Nakayama: "Breeding of Amino Acid Producing Micro-organisms", in: Overproduction of Microbial Products, Krumphanzl, Sikyta, Vanek (eds.), Academic Press, London, UK, 1982) which reduce the production of pantothenic acid.

The microorganisms prepared according to the invention can be cultured continuously or discontinuously in the batch process (batch culture) or in the fed batch (feed process) or repeated fed batch process (repetitive feed process) for the purpose of pantothenic acid production. A summary of known culture methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium to be used must meet the requirements of the particular microorganisms in a suitable manner. Descriptions of culture media for more various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Potassium dihydrogen phosphate or dipotassium hydrogen phosphate or the corresponding sodium-containing salts can be used as the source of phosphorus. The culture medium must furthermore comprise salts of metals, such as e.g. magnesium sulfate or iron sulfate, which are necessary for growth. Finally, essential growth substances, such as amino acids and vitamins, can be employed in addition to the above-mentioned substances. Precursors of pantothenic acid, such as aspartate, β-alanine, ketoisovalerate, ketopantoic acid or pantoic acid, and optionally salts thereof, can moreover be added to the culture medium to additionally increase the pantothenic acid production. The starting substances mentioned can be added to the culture in the form of a single batch, or can be fed in during the culture in a suitable manner.

Basic compounds, such as sodium hydroxide, potassium hydroxide, ammonia, or acid compounds, such as phosphoric acid or sulfuric acid, can be employed in a suitable manner to control the pH of the culture. Antifoams, such as e.g. fatty acid polyglycol esters, can be employed to control the development of foam. Suitable substances having a selective action, e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 20°C to 45°C, and preferably 25°C to 40°C. Culturing is continued until a maximum of pantothenic acid has formed. This target is usually reached within 10 hours to 160 hours.

The concentration of pantothenic acid formed can be determined with known chemical (Velisek; Chromatographic Science 60, 515-560 (1992)) or microbiological methods, such as e.g. the Lactobacillus plantarum test (DIFCO MANUAL, 10^{th} Edition, p. 1100-1102; Michigan, USA).

The following microorganism was deposited on 19th October 1999 as a pure culture at the Deutsche Sammlung für Mikroorganismen und Zellkulturen (DSMZ = German Collection of Microorganisms and Cell Cultures, Braunschweig, Germany) in accordance with the Budapest Treaty:
- Escherichia coli strain DH5α/pCR2.1poxBint as DSM 13114.

The present invention is explained in more detail in the following with the aid of embodiment examples.

### Example 1

### Preparation of a genomic cosmid gene library from Corynebacterium glutamicum ATCC 13032

Chromosomal DNA from C. glutamicum ATCC 13032 was isolated as described by Tauch et al. (1995, Plasmid 33:168-179) and partly cleaved with the restriction enzyme Sau3AI (Amersham Pharmacia, Freiburg, Germany, Product Description Sau3AI, Code no. 27-0913-02). The DNA fragments were dephosphorylated with shrimp alkaline phosphatase (Roche Molecular Biochemicals, Mannheim, Germany, Product Description SAP, Code no. 1758250). The DNA of the cosmid vector SuperCos1 (Wahl et al. (1987) Proceedings of the National Academy of Sciences USA 84:2160-2164), obtained from Stratagene (La Jolla, USA, Product Description SuperCos1 Cosmid Vector Kit, Code no. 251301) was cleaved with the restriction enzyme XbaI (Amersham Pharmacia, Freiburg, Germany, Product Description XbaI, Code no. 27-0948-02) and likewise dephosphorylated with shrimp alkaline phosphatase.

The cosmid DNA was then cleaved with the restriction enzyme BamHI (Amersham Pharmacia, Freiburg, Germany, Product Description BamHI, Code no. 27-0868-04). The cosmid DNA treated in this manner was mixed with the treated ATCC13032 DNA and the batch was treated with T4 DNA ligase (Amersham Pharmacia, Freiburg, Germany, Product Description T4-DNA-Ligase, Code no.27-0870-04). The ligation mixture was then packed in phages with the aid of Gigapack II XL Packing Extract (Stratagene, La Jolla, USA, Product Description Gigapack II XL Packing Extract, Code no. 200217). For infection of the E. coli strain NM554 (Raleigh et al. 1988, Nucleic Acid Res. 16:1563-1575) the cells were taken up in 10 mM MgSO₄ and mixed with an aliquot of the phage suspension. The infection and titering of the cosmid library were carried out as described by Sambrook et al. (1989, Molecular Cloning: A Laboratory Manual, Cold Spring Harbor), the cells being plated out on LB agar (Lennox, 1955, Virology, 1:190) with 100 µg/ml ampicillin. After incubation overnight at 37°C, recombinant individual clones were selected.

### Example 2

### Isolation and sequencing of the poxB gene

The cosmid DNA of an individual colony was isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product No. 27-0913-02). The DNA fragments were dephosphorylated with shrimp alkaline phosphatase (Roche Molecular Biochemicals, Mannheim, Germany, Product Description SAP, Product No. 1758250). After separation by gel electrophoresis, the cosmid fragments in the size range of 1500 to 2000 bp were isolated with the QiaExII Gel Extraction Kit (Product No. 20021, Qiagen, Hilden, Germany).

The DNA of the sequencing vector pZero-1, obtained from Invitrogen (Groningen, The Netherlands, Product Description Zero Background Cloning Kit, Product No. K2500-01) was cleaved with the restriction enzyme BamHI (Amersham Pharmacia, Freiburg, Germany, Product Description BamHI, Product No. 27-0868-04). The ligation of the cosmid fragments in the sequencing vector pZero-1 was carried out as described by Sambrook et al. (1989, Molecular Cloning: A Laboratory Manual, Cold Spring Harbor), the DNA mixture being incubated overnight with T4 ligase (Pharmacia Biotech, Freiburg, Germany). This ligation mixture was then electroporated (Tauch et al., 1994, FEMS Microbiol Letters, 123:343-7) into the E. coli strain DH5αMCR (Grant, 1990, Proceedings of the National Academy of Sciences, U.S.A., 87:4645-4649) and plated out on LB agar (Lennox, 1955, Virology, 1:190) with 50 µg/ml zeocin.

The plasmid preparation of the recombinant clones was carried out with the Biorobot 9600 (Product No. 900200, Qiagen, Hilden, Germany). The sequencing was carried out by the dideoxy chain termination method of Sanger et al. (1977, Proceedings of the National Academies of Sciences, U.S.A., 74:5463-5467) with modifications according to Zimmermann et al. (1990, Nucleic Acids Research, 18:1067). The "RR dRhodamin Terminator Cycle Sequencing Kit" from PE Applied Biosystems (Product No. 403044, Weiterstadt, Germany) was used. The separation by gel electrophoresis and analysis of the sequencing reaction were carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained were then processed using the Staden program package (1986, Nucleic Acids Research, 14:217-231) version 97-0. The individual sequences of the pZero1 derivatives were assembled to a continuous contig. The computer-assisted coding region analyses were prepared with the XNIP program (Staden, 1986, Nucleic Acids Research, 14:217-231).

The resulting nucleotide sequence is shown in SEQ ID No. 1. Analysis of the nucleotide sequence showed an open reading frame of 1737 base pairs, which was called the poxB gene. The poxB gene codes for a polypeptide of 579 amino acids shown in SEQ ID No. 2.

### Example 3

### Preparation of the integration vector pCR2.1poxBint for mutagenesis of the poxB gene

From the strain ATCC 13032, chromosomal DNA was isolated by the method of Eikmanns et al. (Microbiology 140: 1817-1828 (1994)). On the basis of the sequence of the poxB gene known for C. glutamicum from Example 2, the following oligonucleotides were chosen for the polymerase chain reaction:
poxBint1:
poxBint2:

The primers shown were synthesized by MWG Biotech (Ebersberg, Germany) and the PCR reaction was carried out by the standard PCR method of Innis et al. (PCR Protocols. A Guide to Methods and Applications, 1990, Academic Press) with Pwo-Polymerase from Boehringer. With the aid of the polymerase chain reaction, a DNA fragment approx. 0.9 kb in size was isolated, this carrying an internal fragment of the poxB gene and being shown in SEQ ID No. 3.

The amplified DNA fragment was ligated with the TOPO TA Cloning Kit from Invitrogen Corporation (Carlsbad, CA, USA; Catalogue Number K4500-01) in the vector pCR2.1-TOPO (Mead at al. (1991) Bio/Technology 9:657-663). The E. coli strain Top10F' (Grant et al. (1990) Proceedings of the National Academy of Sciences, USA, 87:4645-4649) was then electroporated. Selection for plasmid-carrying cells was made by plating out the transformation batch on LB agar (Sambrook et al., Molecular Cloning: A Laboratory Manual, 2^{nd} Ed., Cold Spring Harbor Laboratory Press, Cold Spring Harbor, N.Y., 1989), which had been supplemented with 50 mg/l kanamycin. Plasmid DNA was isolated from a transformant with the aid of the QIAprep Spin Miniprep Kit from Qiagen and checked by restriction with the restriction enzyme EcoRI and subsequent agarose gel electrophoresis (0.8%). The plasmid was called pCR2.1poxBint (figure 1).

### Example 4

### Preparation of an exchange vector for deletion mutagenesis of the poxB gene

### 4.1 Determination of the nucleotide sequence of the flanks of the poxB gene

In further sequencing steps, the nucleotide sequence of the poxB gene region shown in SEQ ID No. 1 was extended upstream and downstream by in each case approx. 500 to 600 bp. The method described in Example 2 was used for this. The extended nucleotide sequence of the poxB gene region shown in SEQ ID No. 4 was obtained in this manner. The new nucleotide sequence upstream of the poxB gene region shown in SEQ ID No. 1 is shown in SEQ ID No. 6. The new nucleotide sequence downstream of the poxB gene region shown in SEQ ID No. 1 is shown in SEQ ID No. 7.

### 4.2 Construction of a ΔpoxB allele

The method of geneSOEing-PCR described by Horton (Molecular Microbiology 3:93-99 (1995)) was used for construction of the ΔpoxB allele. The primer pairs shown in Table 1 (see also SEQ ID No. 8 to 11) were constructed for this. By means of a PCR, the 5' region before the poxB gene was amplified with primer pair 1 and the 3' region after the poxB gene was amplified with primer pair 2. A further PCR was then carried out with the two amplification products and the primers pox-dell and pox-del4, as a result of which the two amplification products were joined by means of geneSOEing. The deletion fragment or ΔpoxB allele obtained in this way contains the flanking sequences of the poxB gene. The nucleotide sequence of the ΔpoxB allele is shown in SEQ ID No. 12.

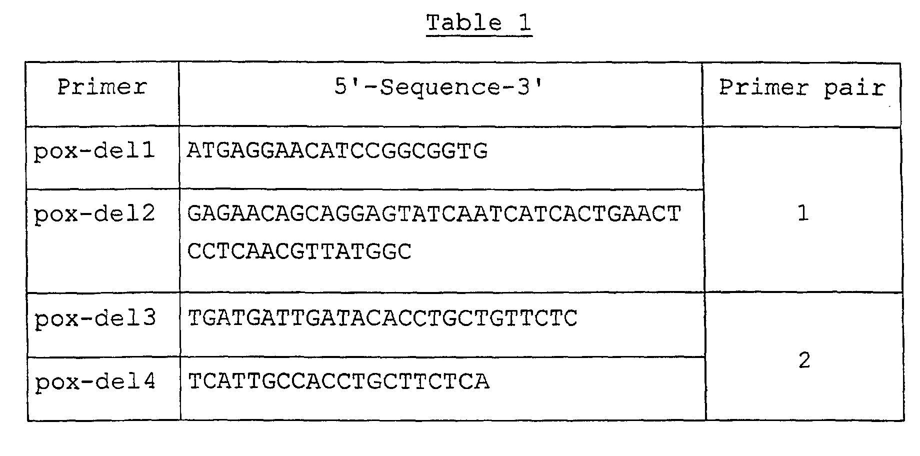

### 4.3 Construction of an exchange vector

The DNA fragment obtained in this way was ligated with the Zero Blunt TOPO PCR Cloning Kit from Invitrogen Corporation (Carlsbad, CA, USA; Catalogue Number K2800-20) in the pCR-Blunt II-TOPO vector (Shuman et al., (1994) Journal of Biological Chemistry 269:32678-32684; Bernard et al., (1983) Journal of Molecular Biology 234:534-541). The oli Stamm Top10 (Grant et al. (1990) Proceedings of the National Academy of Sciences, USA 87:4645-4649) was then transformed with the ligation batch. Selection for plasmid-carrying cells was made by plating out the transformation batch on LB agar (Sambrook et al., Molecular Cloning: A Laboratory Manual, 2^{nd} Ed., Cold Spring Harbor Laboratory Press, Cold Spring Harbor, N.Y.), which had been supplemented with 50 mg/l kanamycin. Plasmid DNA was isolated from a transformant with the aid of the QIAprep Spin Miniprep Kit from Qiagen and checked by restriction with the restriction enzyme EcoRI and subsequent agarose gel electrophoresis (0.8%). The plasmid was called pCRB1-poxBdel (figure 2).

The insert carrying the ΔpoxB allele was excised from this plasmid by means of EcoRI, isolated from the gel and ligated in the non-replicative integration vector pK18mobsacB, which was also cleaved with EcoRI (Schafer et al., Gene 145, 69-73 (1994)). The clonings were carried out in E. coli DH5αmcr (Grant et al., (1990) Proceedings of the National Academy of Sciences, USA, 87: 4645-4649) as the host. The resulting plasmid was called pK18mobsacB-poxBdel.

### Example 5

### Mutagenesis of the poxB gene in the strain FERM BP-1763

The L-valine-producing strain Brevibacterium lactofermentum FERM BP-1763 is described in US-A-5,188,948.

For deletion of the poxB gene, the integration plasmid pKl8mobsacB-poxBdel was electroporated in the strain FERM BP-1763. After selection for kanamycin (25 µg/ml), individual clones in which the inactivation vector was present integrated in the genome were obtained. To allow excision of the vector, individual colonies were incubated in 50 ml liquid LB medium without antibiotics for 24 hours at 30°C and 130 rpm and then smeared on to sucrose-containing agar plates (LB with 15 g/l agar and 10% sucrose). Clones which had lost the vector content again by a second recombination event were obtained by this selection (Jäger et al. 1992, Journal of Bacteriology 174:5462-5465). To identify those clones which carried the ΔpoxB allele, a polymerase chain reaction was carried out with the primers pox-del1 and pox-del4 (Table 1 and SEQ ID No. 8 and 11). These primers amplify on the whole DNA of the starting strain FERM BP-1763 a fragment approx. 3150 bp in size, while on the DNA of poxB deletion mutants the primers amplified a shortened fragment 1422 bp in size. A deletion mutant identified in this way is consequently lacking a region of the poxB gene 1.7 kb in size.

A strain produced and tested in this manner was called FERM BP-1763ΔpoxB and employed for further studies.

### Example 6

### Preparation of pantothenic acid

### 6.1 Production of the strains

The plasmid pND-DBC2, which carries the panB, panC and panD genes of Corynebacterium glutamicum, is known from EP-A-1006192. The plasmid is deposited in the form of the strain ATCC13032/pND-DBC2 as DSM 12437 at the Deutsche Sammlung für Mikroorganismen und Zellkulturen (DSMZ = German Collection of Microorganisms and Cell Cultures, Braunschweig, Germany) in accordance with the Budapest Treaty.

The pantothenic acid-producing strains FERM BP-1763/pND-DBC2 and FERM BP-1763ΔpoxB/pNDDBC2 were formed by transformation of the strains FERM BP-1763 and FERM BP-1763ΔpoxB with the plasmid pND-DBC2.

### 6.2 Preparation of pantothenic acid

In each case a sample of the strains Brevibacterium lactofermentum FERM BP-1763/pND-DBC2 and FERM BP-1763ΔpoxB/pND-DBC2 was smeared on to HHK agar.

HHK agar comprises brain-heart agar, which was obtained from Merck KgaA (Darmstadt, Germany) and supplemented with kanamycin. The composition of the HHK agar is shown in Table 2.

This agar plate culture was incubated for 17 hours at 30ºC and then kept in a refrigerator at +4ºC. Selected individual colonies were then propagated further on the same medium. Cell material of a clone was removed from the HHK agar with an inoculating loop and transferred to 100 mL HHK broth contained in a shaking flask of 1000 mL total volume.

HHK broth comprises brain-heart medium, which was obtained from Merck KgaA (Darmstadt, Germany) and supplemented with glucose and kanamycin. The composition of the HHK broth is shown in Table 3.

**Table 2**

| HHK agar | |
|---|---|
| Substance | Amount per liter |
| Brain-heart agar | 52.0 g |
| Kanamycin | 25 mg |

**Table 3**

| HHK broth | |
|---|---|
| Substance | Amount per liter |
| Brain-heart medium | 37.0 g |
| Kanamycin | 25 mg |
| Glucose | 20.0 g |

The batches were incubated at 30°C and 150 rpm for 22 hours. After the end of the culturing, an optical density of in each case approx. 6 was measured in a photometer at a wavelength of 660 nm (OD 660). This culture of the strain was used to inoculate the production fermenter.

Medium SK-71 shown in Table 4 was used for the fermentation. All the components of the SK-71 medium were initially introduced into the fermenter directly according to the working concentration and sterilized "in situ".

**Table 4**

| Medium SK-71 | |
|---|---|
| Compound | Amount per liter |
| Glucose hydrate | 110.0000g |
| Corn steep liquor (CSL) | 5.0000g |
| β-Alanine | 5.0000g |
| Nicotinic acid | 0.0050g |
| l-Isoleucine | 0.1500g |
| Homoserine | 0.1500g |
| Ammonium sulfate | 25.0000g |
| K dihydrogen phosphate | 0.1000g |
| Mg sulfate 7H₂O | 1.0000g |
| Fe sulfate 7H₂O | 0.0100g |
| Mn sulfate H₂O | 0.0050g |
| CaCl₂ * 2H₂O | 0.0100g |
| Thiamine HCl | 0.0002g |
| D(+)Biotin | 0.0003g |
| Structol | 0.60g |

10 1 stirred reactors from B.Braun (BBI, Germany, Melsungen, Biostat E/ED model) were used as the fermenters.

For inoculation of 1950 g of the fermentation medium SK-71, in each case 100 mL of the shaking flask precultures in HHK broth described above were employed.

The batch was cultured over the entire fermentation time at a temperature of 30°C, a volume-specific aeration of 0.75 vvm, stirring, dependent on the oxygen consumption, of 800 - 1700 rpm and a pH of 7.0 and an oxygen partial pressure of 20% of the atmospheric saturation. The culture was cultured for a total of approx. 49 hours under the above-mentioned conditions until an OD660 of approx. 26 was reached. An aqueous ammonia solution (25 % w/v) was used as the correcting agent for regulating the pH.

The optical density (OD) was then determined with a digital photometer of the type LP1W from Dr. Bruno Lange GmbH (Berlin, Germany) at a measurement wavelength of 660 nm and the concentration of D-pantothenic acid formed was determined by means of HPLC (Hypersil APS 2 5 µm, 250x5 mm, RI detection).

A D-pantothenic acid concentration of approx. 0.20 g/l was measured in the end sample (approx. 49 hours) of the fermentation culture of the strain FERM BP-1763/pND-DBC2. The pantothenic acid concentration in the corresponding sample of the strain FERM BP-1763ΔpoxB/pND-DBC2 was approx. 0.23 g/l.

### Brief Description of the Figures:

- Figure 1: Map of the plasmid pCR2.1poxBint
- Figure 2: Map of the plasmid pCRBl-poxBdel

The base pair numbers stated are approx. values obtained in the context of reproducibility.

The abbreviations and designations used have the following meaning:
Figure 1:
   - ApR: Ampicillin resistance gene
   - ColE1 ori: Replication origin ColE1
   - f1 ori: Replication origin of phage f1
   - KmR: Kanamycin resistance gene
   - lacZ: Residues of the lacZα gene fragment
   - poxBint: Internal fragment of the poxB gene
Figure 2:
   - 'lacZa: 3' end of the lacZα gene fragment
   - 3'-Region: 3' flank of the poxB gene
   - 5'-Region: 5' flank of the poxB gene
   - ccdB: ccdB gene
   - Km: Kanamycin resistance gene
   - lacZa': 5' end of the lacZα gene fragment
   - plac: Promoter of the lac operon
   - pMB1: Replication origin of the plasmid pMB1
   - Zeocin: Zeocin resistance gene

The following abbreviations have moreover been used:
- BamHI:: Cleavage site of the restriction enzyme BamHI
- ClaI: Cleavage site of the restriction enzyme ClaI
- EcoRI:: Cleavage site of the restriction enzyme EcoRI
- HindIII:: Cleavage site of the restriction enzyme HindIII
- SalI:: Cleavage site of the restriction enzyme SalI

## Claims

1. Process for the fermentative preparation of D-pantothenic acid, wherein the following steps are carried out:
a) fermentation of D-pantothenic acid-producing coryneform bacteria in which at least the nucleotide sequence which codes for pyruvate oxidase (EC 1.2.2.2) (poxB) is attenuated, in particular eliminated;
b) concentration of the D-pantothenic acid in the medium or in the cells of the bacteria; and
c) isolation of the D-pantothenic acid produced.

2. Process according to claim 1, wherein the process of insertion, in particular by means of the vector pCR2.lpoxBint, shown in figure 1 and deposited in E. coli as DSM 13114, is used to achieve the attenuation.

3. Process according to claim 1, wherein coryneform bacteria in which further genes of the biosynthesis pathway of D-pantothenic acid are additionally enhanced are employed.

4. Process according to claim 1, wherein coryneform bacteria in which the metabolic pathways which reduce the formation of D-pantothenic acid are at least partly eliminated are employed.

5. Process according to claim 1, wherein coryneform bacteria in which at the same time the panB gene which codes for ketopantoate hydroxymethyl transferase is enhanced are employed.

6. Process according to claim 1, wherein coryneform bacteria in which at the same time the panC gene which codes for pantothenate synthetase is enhanced are employed.

7. Process according to claim 1, wherein coryneform bacteria in which at the same time the ilvC gene which codes for acetohydroxy-acid isomeroreductase is enhanced are employed.

8. Process according to claim 1, wherein coryneform bacteria in which at the same time the ilvD gene which codes for dihydroxy-acid dehydratase is enhanced are employed.

9. Process according to claim 1 or 3, wherein coryneform bacteria in which at the same time the pck gene which codes for phosphoenol pyruvate carboxykinase is attenuated are employed.

10. Process according to claims 6 to 7, wherein the genes mentioned are enhanced in coryneform bacteria which already produce D-pantothenic acid.

11. Process according to claim 8, wherein coryneform bacteria which already produce D-pantothenic acid and in which the pck gene is attenuated are employed.

12. Isolated polynucleotide from coryneform bacteria which lies upstream of SEQ ID No. 1 and is shown in SEQ ID No. 6.

13. Isolated polynucleotide from coryneform bacteria which lies downstream of SEQ ID No. 1 and is shown in SEQ ID No. 7.

14. Isolated polynucleotide from coryneform bacteria, containing a deletion mutation of the poxB gene shown in SEQ ID No. 12.

15. Coryneform bacteria which carry the deletion mutation shown in SEQ ID No. 12.

## Patentansprüche

1. Verfahren zur fermentativen Herstellung von D-Pantothensäure, umfassend die folgenden Schritte:
a) Fermentation von D-Pantothensäure produzierenden coryneformen Bakterien, bei denen wenigstens die Nücleotidsequenz (poxB), die Pyruvatoxidase (EC 1.2.2.2) kodiert, abgeschwächt und insbesondere ausgeschaltet wird;
b) Konzentration der D-Pantothensäure im Medium oder in den Zellen der Bakterien; und
c) Isolierung der produzierten D-Pantothensäure.

2. Verfahren nach Anspruch 1, wobei zur Erzielung der Abschwächung das Insertionsverfahren, insbesondere mit Hilfe des Vektors pCR2.1poxBint, dargestellt in Fig. 1 und hinterlegt in E. coli als DSM 13114, angewendet wird.

3. Verfahren nach Anspruch 1, wobei coryneforme Bakterien verwendet werden, bei denen weitere Gene des Biosyntheseweges der D-Pantothensäure zusätzlich verstärkt werden.

4. Verfahren nach Anspruch 1, wobei coryneforme Bakterien verwendet werden, bei denen die Stoffwechselwege, die die Bildung von D-Pantothensäure verringern, zumindest teilweise ausgeschaltet sind.

5. Verfahren nach Anspruch 1, wobei coryneforme Bakterien verwendet werden, bei denen gleichzeitig das panB-Gen, das Ketopantoat-Hydroxymethyltransferase kodiert, verstärkt wird.

6. Verfahren nach Anspruch 1, wobei coryneforme Bakterien verwendet werden, bei denen gleichzeitig das panC-Gen, das Pantothenatsynthetase kodiert, verstärkt wird.

7. Verfahren nach Anspruch 1, wobei coryneforme Bakterien verwendet werden, bei denen gleichzeitig das ilvC-Gen, das Acetohydroxysäure-Isomeroreduktase kodiert, verstärkt wird.

8. Verfahren nach Anspruch 1, wobei coryneforme Bakterien verwendet werden, bei denen gleichzeitig das ilvD-Gen, das Dihydroxysäure-Dehydratase kodiert, verstärkt wird.

9. Verfahren nach Anspruch 1 oder 3, wobei coryneforme Bakterien verwendet werden, bei denen gleichzeitig das pck-Gen, das Phosphoenolpyruvatcarboxykinase kodiert, abgeschwächt wird.

10. Verfahren nach den Ansprüchen 6 bis 7, wobei die erwähnten Gene in coryneformen Bakterien verstärkt werden, die bereits D-Pantothensäure produzieren.

11. Verfahren nach Anspruch 8, wobei coryneforme Bakterien verwendet werden, die bereits D-Pantothensäure produzieren und bei denen das pck-Gen abgeschwächt wird.

12. Isoliertes Polynucleotid von coryneformen Bakterien, das stromaufwärts der SEQ ID Nr. 1 liegt und in SEQ ID Nr. 6 dargestellt ist.

13. Isoliertes Polynucleotid von coryneformen Bakterien, das stromabwärts der SEQ ID Nr. 1 liegt und in SEQ ID Nr. 7 dargestellt ist.

14. Isoliertes Polynucleotid von coryneformen Bakterien, das eine Deletionsmutation des in SEQ ID Nr. 12 dargestellten poxB-Gens enthält.

15. Coryneforme Bakterien, die die in SEQ ID Nr. 12 dargestellte Deletionsmutation tragen.

## Revendications

1. Procédé pour la préparation fermentative de l'acide D-pantothénique.
**caractérisé par**
les étapes suivantes :
a) fermentation de la bactérie corynebacterium productrice d'acide D-pantothénique dans laquelle au moins la séquence de nucléotide qui code pour la pyruvate oxydase (EC 1 2 2 2) (poxB) est atténuée, en particulier éliminée ;
b) la concentration de l'acide D-pantothénique dans le milieu ou dans les cellules de la bactérie ; et
c) isolation de l'acide D-pantothénique produit.

2. Procédé selon la revendication 1,
dans lequel le procédé d'insertion, en particulier au moyen du vecteur pCR2.1poxBint, représenté sur la figure 1 et déposé dans E.coli sous forme de DSM 13114, est utilisé pour obtenir l'atténuation.

3. Procédé selon la revendication 1,
dans lequel on utilise la bactérie corynebacterium dans laquelle d'autres gènes de la voie de biosynthèse de l'acide D-pantothénique sont de plus améliorés.

4. Procédé selon la revendication 1,
dans lequel on utilise la bactérie corynebacterium dans laquelle les voies métaboliques qui réduisent la formation d'acide D-pantothénique sont au moins partiellement éliminées.

5. Procédé selon la revendication 1, dans lequel on utilise la bactérie corynebacterium dans laquelle en même temps le gène panB qui code pour la kétopantoate hydroxyméthyl transférase est amélioré.

6. Procédé selon la revendication 1,
dans lequel on utilise la bactérie corynebacterium dans laquelle en même temps le gène panC qui code pour la pantothénate synthétase est amélioré.

7. Procédé selon la revendication 1,
dans lequel on utilise la bactérie corynebacterium dans laquelle en même temps le gène ilvC qui code pour l'acétohydroxyacide isoméroréductase est amélioré.

8. Procédé selon la revendication 1,
dans lequel on utilise la bactérie corynebacterium dans laquelle en même temps le gène ilvD qui code pour la dihydroxyacide déshydratase est amélioré.

9. Procédé selon la revendication 1 ou 3,
dans lequel on utilise la bactérie corynebacterium dans laquelle en même temps le gène pck qui code pour la phosphoénolpyruvate carboxykinase est atténué.

10. Procédé selon les revendications 6 à 7,
dans lequel les gènes mentionnés sont améliorés dans la bactérie corynebacterium qui produit déjà l'acide d-pantothénique.

11. Procédé selon la revendication 8.
dans lequel on utilise la bactérie corynebacterium qui produit déjà l'acide D-pantothénique et dans laquelle le gène pck est atténué.

12. Polynucléotide isolé de la bactérie corynebacterium qui se situe en amont de la SEQ ID N° 1 et est montré dans la SEQ ID N° 6.

13. Polynucléotide isolé de la bactérie corynebacterium qui se situe en aval de la SEQ ID N° 1 et est montré dans la SEQ ID N° 7.

14. Polynucléotide isolé à partir de la bactérie corynebacterium, comprenant une mutation de délétion du gène poxB montré dans la SEQ ID N° 12.

15. Bactérie corynebacterium qui porte la mutation de délétion montrée dans la SEQ ID N° 12.
